(19) Europäisches Patentamt
European Patent Office
Office européen des brevets

(11) Veröffentlichungsnummer: **0 248 968**
**A1**

(12) **EUROPÄISCHE PATENTANMELDUNG**

(21) Anmeldenummer: 86810300.3

(22) Anmeldetag: 07.07.86

(51) Int. Cl.³: **C 07 D 213/64**
**A 01 N 43/40**

(30) Priorität: 12.06.86 CH 2376/86

(43) Veröffentlichungstag der Anmeldung: 16.12.87 Patentblatt 87/51

(84) Benannte Vertragsstaaten: AT BE CH DE FR GB IT LI LU NL SE

(71) Anmelder: **CIBA-GEIGY AG**
**Klybeckstrasse 141**
**CH-4002 Basel(CH)**

(72) Erfinder: **Schurter, Rolf, Dr.**
**Holzmattstrasse 45**
**CH-4102 Binningen(CH)**

(54) **(R)-2-]4-(5-Chlor-3-fluorpyridin-2-yloxy)-phenoxy[-propionsäure-propinylester mit herbizider Wirkung.**

(57) Der (R)-2-[4-(5-Chlor-3-fluorpyridin-2-yloxy)-phenoxy]-propionsäurepropinylester zeichnet sich durch aussergewöhnliche herbizide und den Gräserwuchs hemmende Wirkung aus. Er entspricht der Formel I,

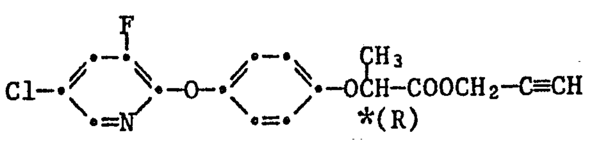

und eignet sich zur selektiven Bekämpfung von Unkräutern in Nutzpflanzenkulturen oder zur Hemmung des Gräserwuchses.

EP 0 248 968 A1

0248968

CIBA-GEIGY AG
Basel (Schweiz)

5-15930/=

# (R)-2-[4-(5-Chlor-3-fluorpyridin-2-yloxy)-phenoxy]-propionsäure-propinylester mit herbizider Wirkung

Die vorliegende Erfindung betrifft den neuen (R)-2-[4-(5-Chlor-3-fluorpyridin-2-yloxy)-phenoxy]-propionsäurepropinylester mit herbizider Wirkung, dessen Herstellung, Mittel welche diesen Ester als Wirkstoff enthalten sowie dessen Verwendung als Herbizid allgemein und speziell zur Bekämpfung von Unkräutern in Nutzpflanzenkulturen wie Getreiden, Reis, Mais, Soja, Zuckerrüben.

Der (R)-2-[4-(5-Chlor-3-fluorpyridin-2-yloxy)-phenoxy]-propionsäure-propinylester entspricht der Formel I

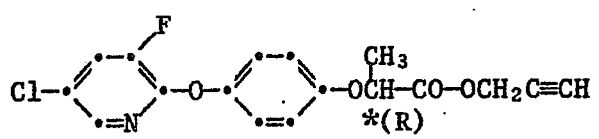

(I).

Der erfindungsgemässe (R)-2-[4-(5-Chlor-3-fluorpyridin-2-yloxy)-phenoxy]-propionsäure-propinylester zeichnet sich durch eine aussergewöhnlich gute Wirkung gegen mono- und einige dikotyle Unkräuter aus, vor allem wirkt er im Nachlaufverfahren gegen widrige in Kulturen wie Getreide, Mais, Reis, Soja und Zuckerrüben vorkommende Unkräuter und Ungräser. Besonders wertvoll ist die Möglichkeit mit ihm Ungräser zu bekämpfen, denen sonst nur schwer beizukonnen ist, wie z.B. Avena fatua, Avena sterilis, Alopecurus myosuroides, Lolium perenne, Phalaris sp., Bromus tectorum, verschiedene Setaria- und Panicum Arten. Die Wirkung tritt unter Feldbedingungen bereits bei niederen Aufwandmengen von weniger als 1 kg pro Hektar auf bei denen die Kulturen nicht oder nur ganz unbedeutend geschädigt werden.

Halogenpyridyloxy-α-phenoxy-propionsäurederivate sind in zahlreichen Publikationen beschrieben worden, siehe beispielsweise die DE-A 2 546 251, 2 649 706, 2 714 622, 2 715 284 sowie die EP-A 483, 1473, 83 556 und 97460. Darin werden α-[4-(3-Fluor-halogenpyridyl-2-oxy)-phenoxy]-propionsäureester zum Teil auch in Erwägung gezogen und vom Umfang miterfasst. Der erfindungsgemässe optisch aktive Ester unterscheidet sich von den bekannten Halogenpyridyloxy-α-phenoxy-propionsäuren durch stärkere Wirkung und damit der Möglichkeit ihn in geringeren Aufwandmengen einzusetzen.

Die Anwendung kann sowohl im Vorauflauf- wie im Nachauflaufverfahren vorgenommmen werden. Dabei können die Aufwandmengen in weiten Grenzen schwanken, z.B. zwischen 0,01 bis 2 kg Wirkstoff pro Hektare.

Ferner besitzt die Verbindung der Formel I günstige wachstumsregulierende Effekte (Wuchshemmung). Insbesondere hemmt sie das Wachstum von Gräsern.

Die Herstellung des neuen Esters der Formel I erfolgt nach verschiedenen Methoden.

Nach einem ersten dieser Verfahren setzt man 5-Chlor-2,3-difluorpyridin der Formel II

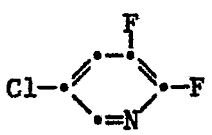

(II),

in einem inerten Lösungs- oder Verdünnungsmittel, in Gegenwart der äquimolaren Menge einer Base mit dem 4-Hydroxyphenoxy-α-propionsäureester der Formel III um,

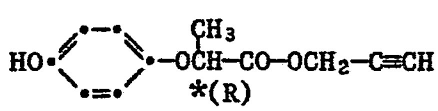

(III).

Ein anderes Verfahren besteht darin, dass man 4-(5-Chlor-3-fluor-pyridin-2-yloxy)-phenol der Formel IV

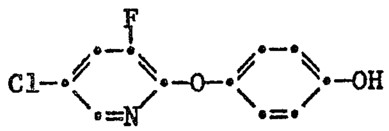

(IV),

in einem inerten Lösungs- oder Verdünnungsmittel in Gegenwart der äquimolaren Menge einer Base mit einem (S)-enantiomeren α-Halogen-propionsäurepropinylester der Formel V umsetzt

$$Hal-\overset{CH_3}{\overset{|}{CH}}-CO-OCH_2-C\equiv CH \qquad (V),$$

worin Hal Chlor, Brom oder Jod bedeutet.

Ein weiteres Verfahren besteht darin, dass man ein (R)-enantiomeres 2-[4-(5-Chlor-3-fluorpyridin-2-yloxy)-phenoxy]-propionsäurehalogenid der Formel VI

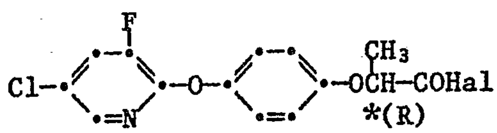

(VI),

worin Hal Fluor, Chlor oder Brom bedeutet, in einem inerten Lösungs- oder Verdünnungsmittel in Gegenwart der äquimolaren Menge einer Base mit Propinol $HOCH_2-C\equiv CH$ umsetzt.

Ferner kann der Ester der Formel I auch hergestellt werden, indem man die (R)-enantiomere 2-[4-(5-Chlor-3-fluorpyridin-2-yloxy)-phenoxy]-propionsäure der Formel VII

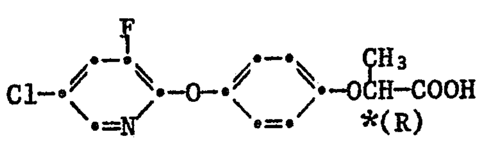

(VII),

in einem inerten Lösungs- oder Verdünnungsmittel, in Anwesenheit der äquimolaren Menge einer Base mit einem Propinylhalogenid der Formel VIII

$$Hal-CH_2-C\equiv CH \qquad (VIII),$$

worin Hal Halogen bedeutet, umsetzt.

Schliesslich besteht ein weiteres Verfahren darin, dass man den (R)-enantiomeren 2-[4-(3-Amino-5-chlorpyridin-2-yloxy)-phenoxy]-propionsäurepropinylester der Formel IX

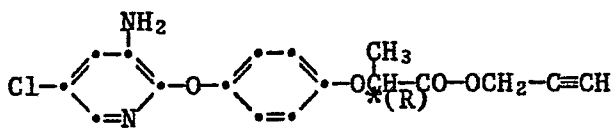

(IX),

nach bekannten Methoden in ein Diazoniumsalz und dieses weiter in die Fluorverbindung überführt.

Gemäss einem anderen Verfahren wird der (R)-enantiomere Ester der Formel I auch hergestellt, indem man in einem inerten organischen Lösungsmittel und in Gegenwart einer Base das 4-(5-Chlor-3-fluor-pyridin-2-yloxy)-phenol der Formel IV

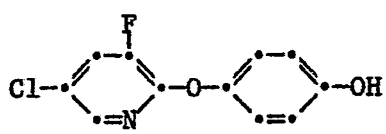

(IV)

mit einem (S)-enantiomeren Milchsäure-propinylester-sulfonat der Formel X umsetzt

$$\begin{array}{c} CH_3 \\ | \\ R-SO_2OCH-CO-OCH_2-C\equiv CH \\ *(S) \end{array} \qquad (X),$$

worin R einen geradkettigen oder verzweigten $C_1$-$C_6$-Alkylrest der unsubstituiert oder durch Halogen, Cyan, $C_1$-$C_4$-Alkoxycarbonyl oder einen Phenylrest, der unsubstituiert oder durch Halogen, $C_1$-$C_4$-Alkyl, $C_1$-$C_4$-Alkoxy, Nitro, Cyan oder $C_1$-$C_4$-Alkoxycarbonyl substituiert ist, bedeutet, und den erhaltenen (R)-2-[4-(5-Chlor-3-fluor-pyridin-2-yloxy)-phenoxy]-propionsäurepropinylester aus dem Reaktionsgemisch isoliert.

Das Ausgangsmaterial der Formel II, 5-Chlor-2,3-difluorpyridin, kann durch Fluorierung des entsprechenden 2,3,5-Trichlorpyridins in Gegenwart von Cäsiumfluorid erhalten werden. Das Produkt wird aus dem Reaktionsgemisch destilliert und durch fraktionierte Destillation gereinigt. Man erhält 5-Chlor-2,3-difluorpyridin auch ausgehend von 2,5-Dichlor-3-nitropyridin, welches man mittels Wasserstoff in Gegenwart von Raney-Nickel-Katalysator zum 3-Amino-2,5-dichlorpyridin reduziert. Das Produkt wird dann mit Natriumnitrit in Fluorwasserstoffsäure zum 2,5-Dichlor-3-fluorpyridin umgewandelt. Diese Verbindung kann mit guter Ausbeute in Gegenwart von Kaliumfluorid zum 5-Chlor-2,3-difluorpyridin fluoriert werden.

4-(5-Chlor-3-fluorpyridyl-2-oxy)-phenol, das Ausgangsprodukt der Formel IV wird durch Kondensation von 5-Chlor-2,3-difluorpyridin mit Hydrochinon, in einem inerten organischen Lösungsmittel in Gegenwart einer Base, hergestellt.

Das Ausgangsmaterial der Formel IX kann durch Reduktion des (R)-2-[4-(5-Chlor-3-nitropyridin-2-yloxy)-phenoxy-propionsäure-propinylesters gewonnen werden.

Die übrigen Ausgangsmaterialien sind entweder bekannt oder können leicht durch konventionelle Synthesen hergestellt werden. Ein Teil dieser Umsetzungen werden vorteilhafterweise in einem den Reaktionspartnern gegenüber inerten, organischen Lösungs- oder Verdünnungsmittel durchgeführt, wie z.B. einem Alkohol, Ester, Aether, Keton, Dimethylformamid, Dimethylsulfoxyd, Acetonitril, einem Aromaten wie Benzol, Toluol etc.

Die Reaktionstemperaturen liegen zwischen -10° und +150°C, praktisch aber zwischen der Raumtemperatur und dem Siedepunkt des Lösungsmittels. Die Reaktionsdauer beträgt je nach dem gewählten Ausgangsstoff, dem Lösungsmittel und der Temperatur 1 Stunde bis ca. einen Tag.

Wo ein Halogenatom bei der Reaktion abgespalten wird, sollte die äquimolare Menge eines säurebindenden Mittels eingesetzt werden. Als solches eignet sich im Prinzip jede anorganische oder organische Base, wie z.B. NaOH, KOH, $NaHCO_3$, $K_2CO_3$, K-tert.Butylat, und Amine, wie Trimethylamin, Triäthylamin, Pyridin, 4-Dimethylaminopyridin etc.

Der neue Wirkstoff der Formel I ist eine stabile Verbindung, welche in üblichen organischen Lösungsmitteln, wie Alkoholen, Aethern, Ketonen, Dimethylformamid, Dimethylsulfoxid etc. löslich sind.

Der Ester der Formel I wird dabei in unveränderter Form oder vorzugsweise zusammen mit den in der Formulierungstechnik üblichen Hilfsmitteln eingesetzt und wird daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen sind die Alkali-, Erdalkali- oder gegebenenfalls substituierten Ammoniumsalze von höheren Fettsäuren ($C_{10}$-$C_{20}$), wie z.B. die Na- oder K-Salze der Oel- oder Stearinsäure, oder von natürlichen Fettsäuregemischen die z.B. aus Kokosnuss- oder Talgöl gewonnen werden können. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkai-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkhol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ringwood, New Jersey, 1979.
Sisley and Wood, "Encyclopedia of Surface Active Agents",
Chemical Publishing Co., Inc. New York, 1964.

Diese Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesonders 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die folgenden Beispiele beschreiben im Detail die Herstellung des erfindungsgemässen (R)-2-[4-(5-Chlor-3-fluorpyridin-2-yloxy)-phenoxy]-propionsäurepropinylesters der Formel I und diesen Ester als Wirkstoff enthaltende Mittel. Die Temperaturen sind in Grad Celsius angegeben, während sich Prozentangaben auf das Gewicht beziehen.

<u>Beispiel 1:</u> Herstellung von (R)-2-[4-(5-Chlor-3-fluorpyridin-2--yloxy)-phenoxy]-propionsäurepropinylester

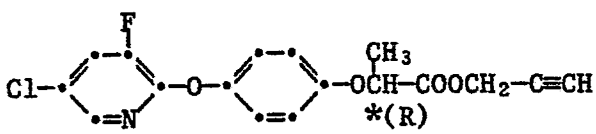

Eine Lösung von 24,2g (0,11 Mol) (R)-2-(4-Hydroxyphenoxy-phenoxy)-propionsäurepropinylester in 80 ml Dimethylformamid wird langsam tropfenweise bei 10°C zu einer gerührten Suspension von 4,9 g (0,11 Mol) Natriumhydrid-Suspension (55%ig) in Dimethylformamid gegeben. Nachdem alles zugetropft ist, wird eine Stunde bei derselben Temperatur weitergerührt. Dann tropft man eine weitere Lösung von 16,4 g (0,10 Mol) 5-Chlor-2,3-difluorpyridin in 40 ml Dimethylformamid zum Reaktionsgemisch und lässt 24 Stunden bei Raumtemperatur rühren. Das Reaktionsgemisch wird dann in Eis/Wasser gegossen und das organische Material dreimal mit Aether extrahiert. Die Aetherphasen werden gesammelt, mit Wasser und Sole gewaschen, über Magnesiumsulfat getrocknet und über Silikagel filtriert. Nach dem Eindampfen des Aether-Filtrates bleibt ein helles Oel zurück, das aus Aethanol kristallisiert wird. Man erhält so 25 g (72 % der Theorie) des (R)-Propinylesters. Schmelzpunkt 55-56°C.

Das als Ausgangsmaterial benötigte 5-Chlor-2,3-difluorpyridin wird folgendermassen hergestellt:

a) 3-Amino-2,5-dichlorpyridin

In eine Lösung von 129,2 g (0,69 Mol) 2,5-Dichlor-3-nitropyridin in 1300 ml Dioxan gibt man 26 g Raney-Nickel, welches vorher mit Aethanol gewaschen wurde. Das Gemisch wird nun mit Wasserstoff bei 20-35°C unter Normaldruck hydriert. Nachdem 20 % der theoretischen Wasserstoffmenge aufgenommen ist, gibt man nochmals 30 g mit Aethanol gewaschenen Raney-Nickel zu. Die Hydrierung wird während 22 Stunden weitergeführt, dann wird der Katalysator abfiltriert, das Lösungsmittel verdampft und der Rückstand aus Hexan/Aethylacetat auskristallisiert. Man erhält so 84,9 g 3-Amino-2,5-dichlorpyridin welches bei 129-132°C schmilzt. Ausbeute 78 % der Theorie.

b) 2,5-Dichlor-3-fluorpyridin

In einem Stahlautoklaven werden 450 ml (22,5 Mol) Fluorwasserstoff vorgelegt. Dazu gibt man unter Rühren bei einer Temperatur von -1°C 163 g (1,0 Mol) 3-Amino-2,5-dichlorpyridin. Dann gibt man langsam bei derselben Temperatur unter Rühren 82,8 g (1,2 Mol) Natriumnitrit in die Lösung. Nachdem alles zugegeben ist, rührt man noch 1,5 Stunden bei einer Temperatur von -5° bis -1°C weiter, lässt dann das Reaktionsgemisch allmählich bis 60°C aufwärmen. Nachdem die Gasentwicklung aufgehört hat, wird der Fluorwasserstoff abdestilliert und der Rückstand in Methylenchlorid aufgenommen. Dann gibt man Eis und Wasser dazu und neutralisiert das Gemisch mit konzentriertem Ammoniak. Die organische Phase wird abgetrennt und die wässrige Phase dreimal mit Methylenchlorid ausgewaschen. Die gesammelten organischen Phasen werden mit Wasser gewaschen, über Magnesiumsulfat getrocknet durch Silikagel filtriert und eingedampft. Auf diese Weise erhält man 141,5 g (85 % der Theorie) 2,5-Dichlor-3-fluorpyridin.

c) 5-Chlor-2,3-difluorpyridin

Eine Suspension von 64,6 g (1,1 Mol) Kaliumfluorid und 11,25 g (0,075 Mol) Cäsiumfluorid in 240 ml Sulfolan (1,1-Dioxo-tetrahydrothiophen) wird vorgelegt. Durch Erwärmen unter vermindertem Druck werden 50 ml Sulfolan wieder abdestilliert. Zur verbleibenden Suspension gibt man unter Rühren eine Lösung von 61,4 g (0,37 Mol)

2,5-Dichlor-3-fluorpyridin in 20 ml Sulfolan. Das Gemisch wird dann während 35 Stunden bei 140°C gerührt. Nach dem Abkühlen giesst man es auf Eis/Wasser und extrahiert das organische Material mit Aether. Die Aetherphase wird mit Wasser gewaschen, über Magnesiumsulfat getrocknet, filtriert und eingedampft. Es verbleiben 48,7 g 5-Chlor-2,3-difluorpyridin als farblose Flüssigkeit welche bei 65-69°/133 mbar siedet. Ausbeute: 88 % der Theorie.

Beispiel 2: Herstellung von (R) (+)-2-[4-(5-Chlor-3-fluorpyridyl-2-oxy)-phenoxy]-propionsäurepropinylester

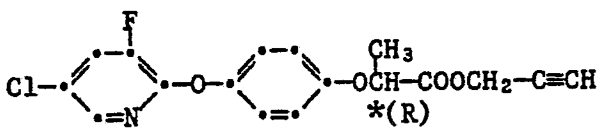

Eine Lösung von 4,9 ml (0,035 Mol) Triethylamin und 2 ml (0,035 Mol) Propinol in 40 ml Toluol wird vorgelegt und mit Eis/Wasser gekühlt. Dann werden 10,6 g (0,030 Mol) (R)-2-[4-(5-Chlor-3-fluorpyridin-2-yloxy)-phenoxy]-propionylchlorid in 30 ml Toluol während 45 Minuten zugetropft. Anschliessend wird 3 Stunden bei Raumtemperatur gerührt. Dann wird das Reaktionsgemisch auf 150 ml Eis/Wasser gegossen und zweimal mit Essigsäure-aethylester extrahiert. Die Extrakte werden mit Sole gewaschen, über Magnesiumsulft getrocknet, filtriert und eingeengt. Der Rückstand wird in Hexan/Aethylacetat 2:1 aufgenommen und zur Reinigung über eine Silikagelsäule chromatographiert. Nach Verdampfen des Lösungsmittels kristallisiert der Rückstand. Die Kristalle schmelzen bei 54°C; $[\alpha]_D^{20}$ +45,4° (2 % in Aceton).

Das als Ausgangsmaterial benötigte (R)-2-[4-(5-Chlor-3-fluorpyridin-2-yloxy)-phenoxy-]-propionsäurechlorid wird folgendermassen hergestellt:

a) 4-(5-Chlor-3-fluorpyridin-2-yloxy)-phenol

Man rührt unter Stickstoffatmosphäre ein Gemisch von 27,5 g (0,25 Mol) Hydrochinon, 11,2 g (0,2 Mol) Kaliumhydroxid und 600 ml Dimethylsulfoxid, bis alles aufgelöst ist. Dann tropft man unter Rühren eine Lösung von 30 g (0,2 Mol) 5-Chlor-2,3-difluorpyridin in

200 ml Dimethylsulfoxid dazu. Die Reaktionslösung wird dann auf 70°C erwärmt und während 4 Stunden bei dieser Temperatur gerührt. Dann giesst man auf Eis/Wasser, stellt das Gemisch mit konzentrierter Salzsäure sauer und extrahiert mit Aethylacetat. Die organischen Phasen werden gewaschen, über Magnesiumsulfat getrocknet, filtriert und zur Trockne eingedampft. Der Rückstand wird in Hexan/Aethylacetat aufgenommen und zur Reinigung über eine Silikagelsäule chromatographiert. Nach Verdampfen des Lösungsmittels kristallisiert der Rückstand. Man erhält 33 g 4-(5-Chlor-3-fluorpyridin-2-yloxy)-phenol als weisse Kristalle, welche bei 97-98°C schmelzen.

b) (R)-2-[4-(5-Chlor-3-fluorpyridin-2-yloxy)-phenoxy]-propionsäuremethylester

Zu einer Suspension von 13,8 g (0,1 Mol) Kaliumcarbonat in 50 ml Dimethylsulfoxid wird tropfenweise unter Rühren eine Lösung von 24,0 g (0,1 Mol) 4-(5-Chlor-3-fluorpyridin-2-yloxy)-phenol in 80 ml Dimethylsulfoxid gegeben und das Gemisch während 2 Stunden bei Raumtemperatur gerührt. Dann tropft man innerhalb 30 Minuten eine Lösung von 25,8 g (0,1 Mol) S(-)-Milchsäuremethylester-tosylat in 30 ml Dimethylsulfoxid zu. Anschliessend wird das Reaktionsgemisch während 20 Stunden bei 60°C gerührt. Zur Aufarbeitung wird es auf Eis/Wasser gegossen und dreimal mit Aether extrahiert. Die Extrakte werden mit Wasser und Sole gewaschen, über Magnesiumsulfat getrocknet, filtriert und eingeengt. Das eraltene Rohprodukt wird durch Chromatographie an Kieselgel gereinigt (Lösungsmittelgemisch: Hexan/Essigsäureaethylester 3 :1). Man erhält so 26,0 g reines Produkt als Oel ($[\alpha]_D^{20}$= +38,8±0,5° in Aceton).

c) (R) (+)-2-[4-(5-Chlor-3-fluorpyridin-2-yloxy)-phenoxy]-propionsäure

13,0 g (0,040 Mol) (R) (+)-2-[4-(5-Chlor-3-fluorpyridin-2-yloxy)-phenoxy]-propionsäure-methylester werden in 65 ml Dioxan gelöst. Dann gibt man unter Rühren 42 ml 1N Natronlauge zu und erwärmt die Lösung 2,5 Stunden auf 35°C. Anschliessend wird das Reaktionsgemisch auf Eis/Wasser gegossen und mit 22 ml 2N Salzsäure angesäuert. Darauf wird zweimal mit Aethylacetat extrahiert, die Extrakte mit

Sole gewaschen, über Magnesiumsulfat getrocknet, filtriert und eingeengt. Der Rückstand wird aus Essigsäureaethylester/Hexan kristalllisiert. Man erhält so 10,2 g weisse Kristalle mit Schmelzpunkt 95-96°C. Ausbeute 81,8 % der Theorie. $[\alpha]_D^{20}$ +37,7±0,5° in Aceton.

d) (R)-2-[4-(5-Chlor-3-fluorpyridin-2-yloxy)-phenoxy]-propionsäurechlorid

10,8 (0,035 Mol) (R)-2-[4-(5-Chlor-3-fluorpyridin-2-yloxy)-phenoxy]-propionsäure werden in 150 ml Toluol gelöst. Mit einem Bad von 130°C werden 50 ml Toluol abdestilliert. Anschliessend wird das Gemisch auf eine Innentemperatur von 90°C gekühlt und 3,8 ml Thionylchlorid werden langsam unter Rühren zugetropft. Das Reaktionsgemisch wird 16 Stunden bei der gleichen Temperatur weitergerührt und anschliessend unter Vakuum eingeengt. Das so erhaltene Säurechlorid, Schmelzpunkt von 47-48°C, wird direkt weiterverwendet.

Beispiel 3: Herstellung einer Formulierung mit flüssigen Wirkstoffen der Formel I (% = Gewichtsprozent)

| Emulsions-Konzetrate | a) | b) | c) |
|---|---|---|---|
| (R)-2-[4-(5-Chlor-3-fluorpyridin--2-yloxy)-phenoxy]-propionsäurepropinylester | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenoyl-polyäthylenglykoläther (30 Mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| Lösungen | a) | b) | c) | d) |
|---|---|---|---|---|
| Propinylester (wie oben) | 80 % | 10 % | 5 % | 95 % |
| Aethylenglykol-monomethyl-äther | 20% | - | - | - |
| Polyäthylenglykol M G 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenze 160-190°C) | - | - | 94 % | - |

Die Lösungen sind zur Anwendung in Form kleinster Tropfen geeignet.

| Granulate | a) | b) |
|---|---|---|
| (R)-2-(5-Chlor-3-fluorpyridin-2-yloxy)-phenoxy]-propionsäurepropinylester | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| Stäubemittel | a) | b) |
|---|---|---|
| Propinylester | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| a) Spritzpulver | a) | b) |
|---|---|---|
| Propinylester | 20 % | 60 % |
| Na-Ligninsulfonat | 5 % | 5 % |
| Na-Laurylsulfat | 3 % | - |
| Na-Diisobutylnaphthalinsulfonat | - | 6 % |
| Octylphenolpolyäthylenglykoläther (7-8 Mol AeO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| (R)-2-[4-(5-Chlor-3-fluorpyridin-2-yloxy)-phenoxy]-propionsäurepropinylester | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Propinylester | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

<u>Extruder-Granulat</u>

| | |
|---|---|
| Propinylester | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

<u>Umhüllungs-Granulat</u>

| | |
|---|---|
| Propinylester | 3 % |
| Polyäthylenglykol (M G 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

<u>Suspensions-Konzentrat</u>

| | |
|---|---|
| (R)-2-[4-(5-Chlor-3-fluorpyridin-2-yloxy)-phenoxy]-propionsäurepropinylester | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 5: Herbizide Wirkung

Post-emergente Herbizid-Wirkung (Kontaktherbizid)

Die Grasarten Zea maïs, Sorghum hybridum, Oryza sativa, Avena fatua, Bromus tectorum, Lolium perenne, Alopecurus myosuroïdes, Digitaria sanguinalis, Echinochloa crus galli, Sorghum halepense und Rottboellia exaltata wurden im Gewächshaus in Töpfen gezogen und nach dem Auflaufen (im 4- bis 6-Blattstadium) mit einer wässerigen Dispersion des erfindungsgemässen (R)-Enantiomers und des Racemates in verschiedenen Dosierungen, ausgedrückt in g Wirk-substanz pro Hektare, auf die Pflanzen gespritzt und diese bei einer Temperatur von 24-26°C bei 45-60 % relativer Luftfeuchtigkeit und regelmässigem Bewässern gehalten. Der Versuch wird 10 Tage nach der Behandlung ausgewertet. Der an den Pflanzen angerichtete Schaden wird gemäss einer 9er Skala bonitiert:

Pflanze

9 % gedeiht wie unbehandelte Kontrollpflanze
1 % Totalschaden, Pflanze abgestorben
2-8 % Zwischenstufen der Schädigung.

Die Bonitur wird für jede Aufwandmenge aus den geprüften Grasarten ermittelt und die Mittelwerte in der beiliegenden Graphik eingetragen.

Darin ist die durchschnittliche Nachauflaufaktivität der beiden geprüften Wirkstoffe gegen die Gräser dargestellt. Wie aus der Abbildung ersichtlich, wird zur Erreichung eines Boniturwertes von 3, welcher einer guten Herbizidwirkung entspricht, eine Aufwandmenge von 86 g AS/ha benötigt, wenn das Racemat verwendet wird. Der gleiche Bekämpfungsgrad wird überraschenderweise bereits mit 42 g AS/ha erreicht, wenn das R-Enantiomere eingesetzt wird. Daraus ergibt sich eine unerwartete Aktivitätssteigerung um mehr als den Faktor 2.

Patentansprüche

1. (R)-2-[4-(5-Chlor-3-fluorpyridin-2-yloxy)-phenoxy]-propionsäure-propinylester der Formel I

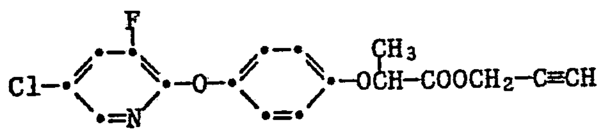

(I).

2. Herbizides und das Pflanzenwachstum hemmendes Mittel, dadurch gekennzeichnet, dass es als Wirkstoff den (R)-2-[4-(5-Chlor-3-fluor-pyridin-2-yloxy)-phenoxy]-propionsäurepropinylester der Formel I, Anspruch 1, enthält, zusammen mit inerten Zutaten.

3. Die Verwendung des (R)-2-[4-(5-Chlor-3-fluorpyridin-2-yloxy)-phenoxy]-propionsäurepropinylesters der Formel I, Anspruch 1 oder ein ihn enthaltendes Mittel, als selektives Unkrautmittel in Kulturpflanzungen.

4. Verfahren zur Herstellung des (R)-2-[4-(5-Chlor-3-fluorpyridin-2-yloxy)-phenoxy]-propionsäurepropinylester gemäss Anspruch 1, dadurch gekennzeichnet, dass man 5-Chlor-2,3-difluorpyridin der Formel II

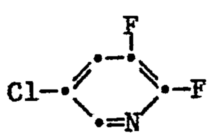

(II),

in einem inerten Lösungs- oder Verdünnungsmittel, in Gegenwart der äquimolaren Menge einer Base mit dem 4-Hydroxyphenoxy-α-propion-säureester der Formel III umsetzt,

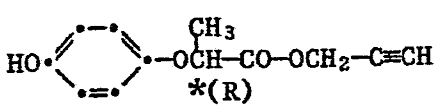

(III).

5. Verfahren zur Herstellung des (R)-2-[4-(5-Chlor-3-fluorpyridin-2-yloxy)-phenoxy]-propionsäurepropinylester gemäss Anspruch 1, dadurch gekennzeichnet, dass man ein (R)-enantiomeres 2-[4-(5-Chlor-3-fluorpyridin-2-yloxy)-phenoxy]-propionsäurehalogenid der Formel VI

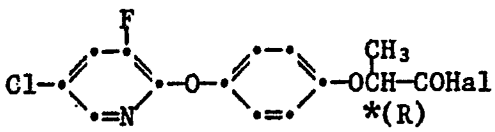

(VI),

worin Hal Fluor, Chlor oder Brom bedeutet, in einem inerten Lösungs- oder Verdünnungsmittel in Gegenwart der äquimolaren Menge einer Base mit Propinol, $HOCH_2-C{\equiv}CH$ umsetzt.

FO 7.5/NU/cw*

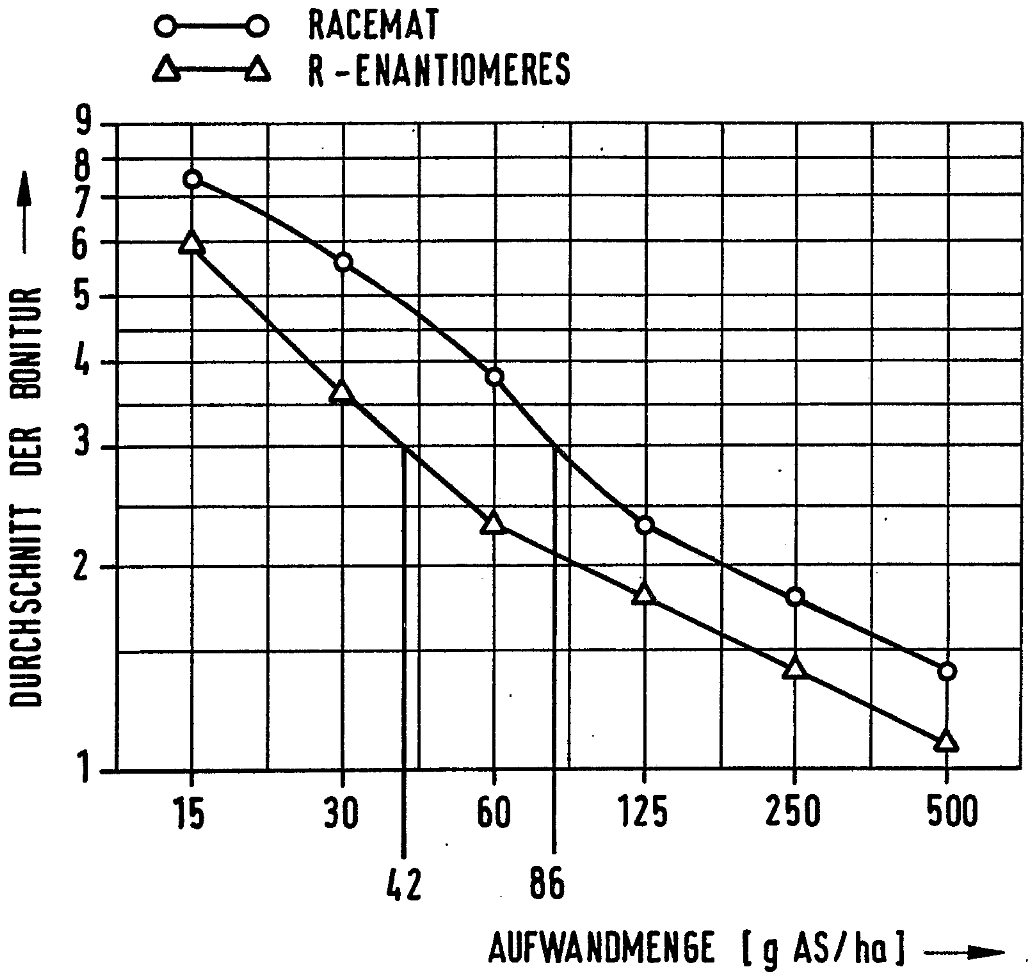
RACEMAT
R-ENANTIOMERES
DURCHSCHNITT DER BONITUR
9
8
7
6
5
4
3
2
1
15
30
60
125
250
500
42
86
AUFWANDMENGE [g AS/ha]

Europäisches Patentamt

# EUROPÄISCHER RECHERCHENBERICHT

Nummer der Anmeldung
0248968
EP 86 81 0300

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 083 556 (CIBA-GEIGY)<br>* Ansprüche 1,7,9,12,14 *<br>--- | 1-5 | C 07 D 213/64<br>A 01 N 43/40 |
| Y | EP-A-0 151 429 (BAYER)<br>* Ansprüche 1,3,4,6 *<br>--- | 1-3,5 | |
| Y | EP-A-0 002 800 (HOECHST)<br>* Ansprüche 1,3-4 *<br>--- | 1-3 | |
| Y | EP-A-0 133 033 (ICI)<br>* Anspruch 8 *<br>--- | 4 | |
| A | EP-A-0 006 608 (CIBA-GEIGY)<br>* Ansprüche 1,9-10 *<br>--- | 1-3 | |
| E | EP-A-0 191 736 (CIBA-GEIGY)<br>* Seite 63, Tabelle 2: Verbindung Nr. 2-19 *<br>----- | 1 | RECHERCHIERTE SACHGEBIETE (Int. Cl.4)<br>C 07 D 213/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 21-04-1987 | SCARPONI U. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument